# EUROPEAN PATENT APPLICATION

(11) **EP 4 364 792 A1**
(43) Date of publication of application: **08.05.2024**
(21) Application number: 22204941.3
(22) Date of filing: 01.11.2022
(51) Int. Cl.: A61N 5/06

(54) **SCALP TREATMENT DEVICE**

(71) Applicant: Koninklijke Philips N.V., 5656 AG Eindhoven (NL)
(72) Inventor: LELIEVELD, Mark Johannes, Eindhoven (NL); LAM, HIU MAN, Eindhoven (NL); NUIJS, Antonius Maarten, 5656AG Eindhoven (NL); CHEUNG, Kit Man Lisa, 5656AG Eindhoven (NL); BROWN, Guy Anthony, 5656 AG Eindhoven (NL); BUT, Kam Wah Anthany, 5656 AG Eindhoven (NL)
(74) Representative: Philips Intellectual Property & Standards

(57) **Abstract**

A scalp treatment device comprises a light source for generating visible light and a head with an array of bristles. At least some of the bristles comprise a light guide for delivering light from the light source to the scalp. In a pre-treatment mode, the light source is controlled to display information to the user by means of a pattern of illuminated bristles with a first intensity whereas in a treatment mode, the light source is controlled to provide treatment light with a second intensity to the bristles, higher than the first intensity. This device uses bristles which emit light both as treatment (functional) elements and also as user interface (light output) elements. This reduces the weight and complexity of the device.

## Description

### FIELD OF THE INVENTION

This invention relates to scalp treatment devices.

### BACKGROUND OF THE INVENTION

Approximately 40% of adults suffer from persistent flakes on the scalp. These flakes can originate from dandruff, dry scalp skin or from inflammatory skin diseases like seborrheic dermatitis and psoriasis.

The flakes are visible on the scalp and the hair and they shed on the shoulders, which poses an issue for many of those affected. In all cases of flakes, the epidermis of the skin is perturbed, with a high turnover rate and incomplete differentiation of keratinocytes, which causes the resulting corneocytes to shed not as separate cells but as clumps, known as flakes.

In the case of dandruff, the underlying cause is oily scalp in combination with the overpopulation of a commensal fungus known as Malassezia which feeds on sebum, and produces free fatty acids that irritate the scalp. This induces an immune response leading to aforementioned perturbation of the epidermis.

In the case of flakes from dry scalp, the low level of sebum cannot prevent loss of moisture from the epidermis, resulting in a compromised barrier function, an immune response and a similar perturbation of the epidermis.

Finally, in the case of psoriasis, seborrheic dermatitis and various other skin diseases, the underlying inflammation is the cause of the perturbation of skin physiology associated with scales and flakes.

In practice, people suffering from ordinary dandruff or flakes from a dry scalp can only use anti-dandruff or moisturizing shampoos. The first type limits proliferation of Malassezia fungus while the second type delivers moisturizing ingredients to the scalp skin, thus following separate approaches to deal with flakes.

The two main disadvantages of these shampoos are firstly that they only provide a shortlasting relief from the flakes, typically a couple of days and secondly that consumers are generally unable to distinguish between dry scalp flakes and dandruff and consequently often choose the wrong type of shampoo.

Light-based devices for the treatment and prevention of flakes have been proposed. UV-A and UV-B devices have for example been proposed, for use by professionals for the treatment of skin diseases on the scalp. Devices have also been proposed for use on the scalp operating with visible light. For example, yellow light (590 ± 5 nm) has been proposed for the treatment of dandruff and blue light (470 ± 5 nm) has been proposed for prevention of proliferation of micro-organisms. Red light (at 660 nm) and UV/violet light (at 395 nm) have also been proposed for the treatment of seborrheic dermatitis on the scalp.

These light-based devices are not generally available to consumers.

Devices based on UV light should only be used by professionals and only for treatment of skin diseases on the scalp like psoriasis and seborrheic dermatitis. The use of UV for the treatment of ordinary dandruff and flakes from dry skin is not a sensible option as the risk of side effects like skin cancer likely outweighs the benefits.

There is thus a need for a solution a consumer can use at home, that is safe and preferably effective on both dandruff and flakes from dry scalp and that provides relief from flakes for a longer time than just a couple of days.

For a consumer device, it must be easy to use and lightweight. In particular, the device should enable feedback to be provided to a user to assist in the use of the device, but without making the device too heavy or expensive.

### SUMMARY OF THE INVENTION

The invention is defined by the claims.

According to examples in accordance with an aspect of the invention, there is provided a scalp treatment device, comprising:
a light source for generating visible light;
a head with an array of bristles, wherein at least some of the bristles comprise a light guide having a proximal end at which the light guide attaches to the head and a distal end, wherein the light guide is for delivering light from the light source to the scalp from the distal end; and
a controller for controlling the light delivery to the light guides,
wherein the controller is configured:
   in a pre-treatment mode, to control the light source to display information to the user by means of a pattern of illuminated bristles with a first intensity;
   in a treatment mode, to control the light source to provide treatment light with a second intensity to the bristles, higher than the first intensity.

This device uses bristles which emit light (by means of a light guide structure) both as treatment (functional) elements and also as user interface (light output) elements. The light output function makes use of lower intensity light before the high intensity light treatment. This reduces the risk of the user looking at the bristles when the high intensity light output is being generated.

The visible light is for use on the human scalp, for the treatment of recurring flakes from dandruff, dry scalp or skin diseases causing flakes, such as seborrheic dermatitis and psoriasis.

The use of the bristles as a user interface as well as functional treatment elements reduces the weight of the device. This means the device can be more easily mounted to the head (without falling off) while the treatment is performed, or it can more easily be held by the user. The need for a separate user interface is avoided, thus reducing cost and weight.

The information for example comprises a timing count down to the treatment mode, and the pattern comprises a sequence of numbers. Thus, the user can be advised when the high intensity light treatment will begin.

The information may comprise, or further comprise, a warning that the treatment mode is imminent and the pattern comprises illumination of all light guides (with the lower intensity).

When these two types of information are combined, the user is given a count down to the treatment, and an initial period which warns the user that the treatment is about to start. The warning for example has a fixed duration such as 5 seconds.

The information may further comprise initial device status information. For example, when the device is turned on, for a battery operated device, the initial device status information may comprise a battery level.

The controller may be configured to apply the treatment mode for a predetermined treatment time. This may be a default time period or a user-settable time period.

An indication may be provided when treatment is over.

The light source for example comprises a blue light source with peak output intensity at a wavelength in the range 400nm to 470nm.

The light source for example comprises an array of LEDs, with one LED or a sub-array of LEDs associated with each bristle. Each bristle then has independently controllable light output so that any pattern may be generated.

The device may take the form of a treatment hairbrush with a head and a handle, and the treatment is similar to brushing the hair, with the bristles in contact with the scalp and moved across the scalp area. The light source is then located in a head of the device.

The device may instead take the form of a cap to be worn by a user.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a scalp treatment device;
Fig. 2 shows different light output patterns provided to the bristles of the scalp treatment device;
Fig. 3 shows a scalp treatment system in more detail; and
Fig. 4 shows the cap part of the system of Fig. 3 in more detail.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a scalp treatment device which comprises a light source for generating visible light and a head with an array of bristles. At least some of the bristles comprise a light guide for delivering light from the light source to the scalp. In a pre-treatment mode, the light source is controlled to display information to the user by means of a pattern of illuminated bristles with a first intensity whereas in a treatment mode, the light source is controlled to provide treatment light with a second intensity to the bristles, higher than the first intensity. This device uses bristles which emit light both as treatment (functional) elements and also as user interface (light output) elements. This reduces the weight and complexity of the device.

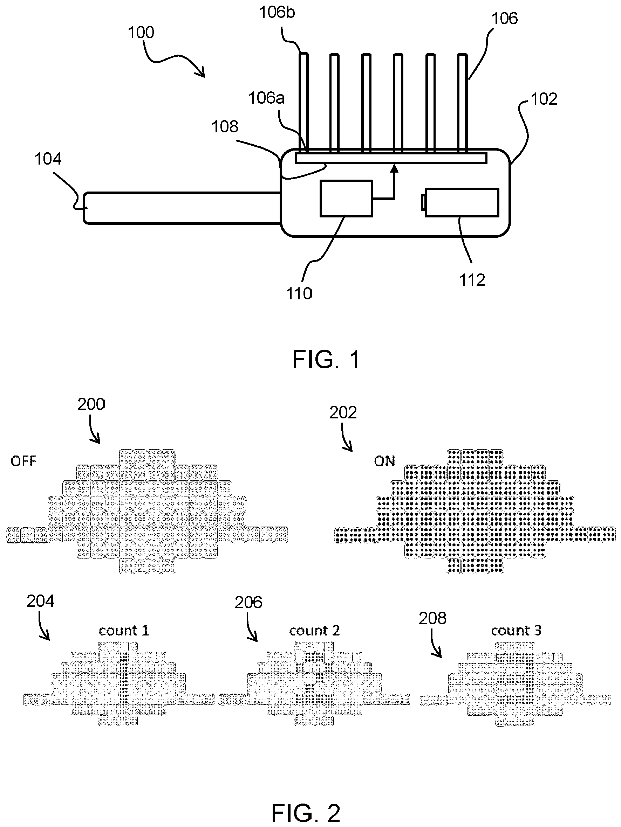

Fig. 1 shows a scalp treatment device 100 with the general form of a hairbrush.

The device comprises a head 102 and a handle 104. The head 102 carries an array of bristles 106. The bristles have a proximal end where they are fixed to the head 102 and a remote free end. The fixed proximal end 106a and free distal end 106b of one bristle is labelled in Fig. 1. The bristles 106 connect to (or are positioned adjacent to) a light source 108 within the head. The light source may in another example be in the handle, with light coupling from the handle to the head.

The bristles each comprise a light guide. At the proximal end of the light guide, the coupling of the bristle to the light source provides a coupling of light from the light source into the light guide. Light is then guided along the light guide and escapes at the free end. In use, the free end is intended to contact the scalp, i.e. the bristles are positioned and/or moved through the hair. The light guide typically guides light using total internal reflection from the fixed end to the free end. However, an outer surface of each light guide may instead or additionally be reflective to provide the containment of light inside the light guide until it reaches the free end.

In another arrangement, the device is in the form of a cap to be worn by the user, with the bristles. The device is then in the form of a head alone, without the need for a handle.

The treatment is for example intended for a predetermined treatment time. This may be a default time period or a user-settable time period. Typically, an area on the scalp should receive light for a period of 2 to 30 minutes, depending on the level of irradiance. The treatment can be performed with a brush, but for a long treatment procedure for the whole scalp, a worn cap will be more convenient for the user.

The device has a controller 110 for controlling the light source, and hence for controlling the light delivery to the light guides. The device is typically battery-operated and a battery 112 is shown, although it may also be a mains-operated device.

The light source comprises an array of LEDs. One LED or a sub-array of LEDs is positioned at a location to inject light into a respective one of the light guides. The LEDs are for example blue light LEDs with peak output intensity at a wavelength in the range 400nm to 470nm. However, as mentioned above, red and yellow light have also been proposed. The configuration of the device may be used with any desired light wavelength, or multiple wavelengths of light. Different bristles may deliver different wavelengths, or multiple wavelengths may be provided to each bristle.

For the example of a hairbrush, there may be additional bristles to the lightguide bristles, and these additional bristles may improve the hair brushing function.

The light source is controllable to deliver light of different intensities. Thus, as a minimum, there is a first intensity for providing visible light at an intensity suitable to function as a display function to a user. There is a second intensity, higher than the first intensity, to function as a treatment light. The intensity may for example be higher than might be comfortable for a user to look at.

By way of example, the intensity for treatment may typically be between 0.1 and 200 mW/cm², and preferably between 10 and 100 mW/cm² . If the light is pulsed with a certain duty cycle, the time-averaged intensity can lie between 0.1 and 200 mW/cm², but then the peak irradiance will be higher.

In display mode the intensity will be much lower than 200 mW/cm² since such high values are not comfortable for viewing and can even be dangerous.

There may be more intensity settings than only two.

The controller is configured to control the light source to display information to the user by means of a pattern of illuminated bristles with the first intensity. This is used as a pre-treatment mode. The information conveyed is at least that the device is active and a treatment mode is about to commence.

The controller is then configured, in the treatment mode, to control the light source to provide treatment the light with the second intensity.

The use of the bristles as a user interface as well as functional treatment elements reduces the weight of the device. In the case of a cap, this means it can be more easily mounted to the head (without falling off) while the treatment is performed, or in the case of a brush it can more easily be held by the user.

Fig. 2 shows examples of possible patterns of light provided by the light source. It shows the pattern made by the ends of the bristles for an example of a cap. Thus, the shape represents the shape of the bristle locations within a flattened out cap. When worn, the bristle locations match the contour of the scalp.

The bristles are shown as square cross section light guides, and the LEDs which feed light into the light guides are shown as dots.

Pattern 200 has all light sources turned off.

Pattern 202 has all light sources turned on, for example to the full treatment intensity.

Patterns 204, 206, 208 show a display image being provided, with the bristles functioning as pixels of the display. In particular, pattern 204 shows a "1", pattern 206 shows a "208" and pattern 204 shows a "3". For this display information, the light source is operated with the lower, first, intensity.

These patterns each convey display information rather than having a treatment function. Thus, there are intended to be seen by the view before the device is applied to the scalp, to provide information to the user before the treatment begins.

The numbers of patterns 204, 206 and 208 may be used to provide a timing count down from the pre-treatment mode to the treatment mode. Thus, the user can be given a count down (3-2-1) to when the high intensity light treatment will begin.

At the end of the count down, there may also be the pattern 202 with illumination of all bristles (but still with the low intensity) warning that the treatment mode is now imminent. This may have a fixed duration such as 5 seconds.

In this way, the user is given a count down to the treatment, and an initial period which warns the user that the treatment is about to start. This gives the user time to position the device correctly.

The display function may be used to provide other information than the timing information explained above. For example, when the device is turned on, initial device status information may be provided such as a brand logo, a battery level, self-diagnostics information (e.g. an error code for any device error) etc.

The device may also indicate to the user when the treatment is over. This may simply because the device has shut off, and the user will notice this as a result of the disappearance of scattered light or light leakage.

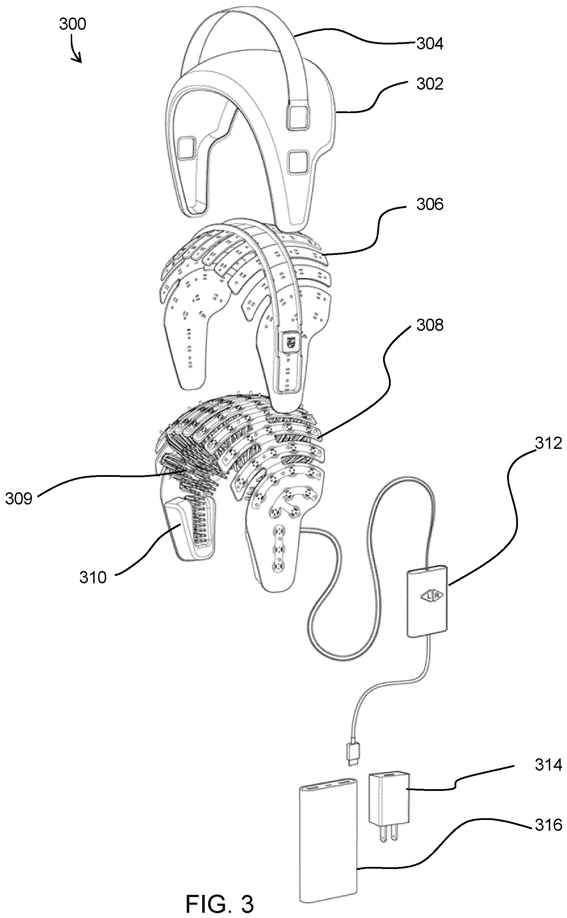

Fig. 3 shows a scalp treatment system 300 in more detail. The system has a removable outer cover 302 for example formed as a fabric foam laminate, with a handle 304 in the form of an elastic strap.

The cap comprises an outer support frame 306, for example formed of nylon, and an inner frame 308 which supports the lightguides 309. The inner frame 308 is for example a silicone body which defines the lightguides, and and it incorporates an integrated PCB which carries the LED light sources. Ear cushions 310 are provided over the ears formed of a soft foam with synthetic covers.

A control box 312 functions as the user interface of the device (as well as incorporating the controller). The device is powered by a USB charger 314 or an external power bank 316.

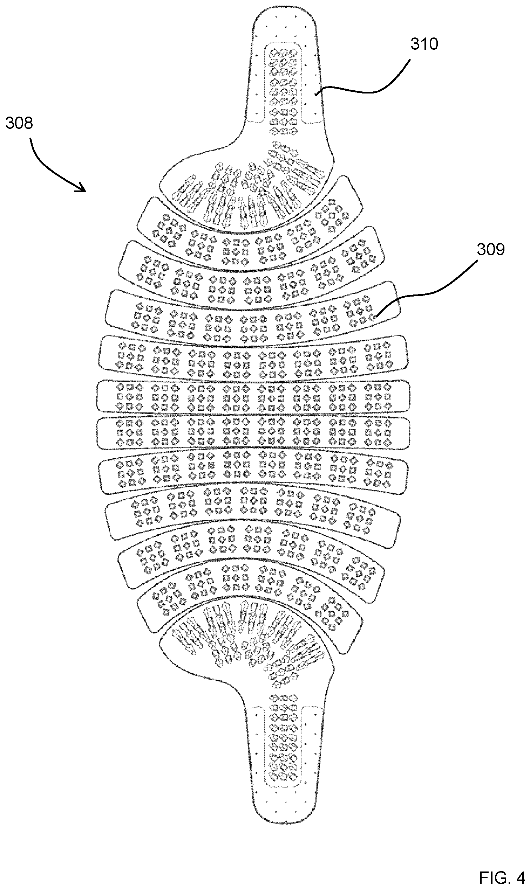

Fig. 4 shows the inner frame part 308 of the system of Fig. 3 in more detail, again in a flattened out view.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A scalp treatment device (100), comprising:
a light source (108) for generating visible light;
a head (102) with an array of bristles (106), wherein at least some of the bristles comprise a light guide having a proximal end (106a) at which the light guide attaches to the head and a distal end (106b), wherein the light guide is for delivering light from the light source to the scalp from the distal end; and
a controller (110) for controlling the light delivery to the light guides,
wherein the controller is configured:
in a pre-treatment mode, to control the light source to display information to the user by means of a pattern (104, 206, 208) of illuminated bristles with a first intensity;
in a treatment mode, to control the light source to provide treatment light with a second intensity to the bristles, higher than the first intensity.

2. The device of claim 1, wherein the information comprises a timing count down to the treatment mode, and the pattern comprises a sequence of numbers.

3. The device of claim 1 or 2, wherein the information comprises, or further comprises, a warning that the treatment mode is imminent and the pattern comprises illumination of all light guides.

4. The device of any one of claims 1 to 3, wherein the information further comprises initial device status information.

5. The device of claim 4, wherein the device is battery operated, and the initial device status information comprises a battery level.

6. The device of any one of claims 1 to 5, wherein the controller is configured to apply the treatment mode for a predetermined treatment time.

7. The device of any one of claims 1 to 6, wherein the light source comprises a blue light source with peak output intensity at a wavelength in the range 400nm to 470nm.

8. The device of claim 7, wherein the light source comprises an array of LEDs, with one LED or a sub-array of LEDs, associated with each bristle.

9. The device of any one of claims 1 to 8, comprising a brush with a head and a handle, wherein the light source is located in the head.

10. The device of any one of claims 1 to 9, comprising a cap to be worn by a user.
